(19) Europäisches Patentamt
European Patent Office
Office européen des brevets

(11) Publication number: **0 067 580**
**B1**

(12) **EUROPEAN PATENT SPECIFICATION**

(45) Date of publication of patent specification: **15.01.86**

(51) Int. Cl.$^4$: **C 10 J 3/54,** C 07 C 9/04, C 07 C 1/06

(21) Application number: **82302732.1**

(22) Date of filing: **27.05.82**

(54) **An integrated catalytic coal devolatilisation and steam gasification process.**

(30) Priority: **05.06.81 US 270739**

(43) Date of publication of application:
**22.12.82 Bulletin 82/51**

(45) Publication of the grant of the patent:
**15.01.86 Bulletin 86/03**

(84) Designated Contracting States:
**BE DE FR GB NL**

(56) References cited:
**EP-A-0 008 469**
**FR-A-2 381 820**
**US-A-2 634 286**
**US-A-2 694 624**
**US-A-3 004 839**
**US-A-3 929 431**
**US-A-4 292 048**

(73) Proprietor: **Exxon Research and Engineering Company**
**P.O.Box 390 180 Park Avenue**
**Florham Park New Jersey 07932 (US)**

(72) Inventor: **Lamb, Charles Wendell**
**5003 Cedar Creek**
**Dickinson, Texas (US)**

(74) Representative: **Field, Roger Norton et al**
**ESSO Engineering (Europe) Ltd. Patents & Licences Apex Tower High Street**
**New Malden Surrey KT3 4DJ (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).



Courier Press, Leamington Spa, England.

**Description**

This invention relates to the devolatilization and gasification of coal and similar carbonaceous materials and is particularly concerned with an integrated catalytic devolatilization and steam gasification process carried out in the presence of a carbon-alkali metal catalyst to produce either a methane-containing gas or a combination of a methane-containing gas and hydrocarbon liquids.

Existing and proposed processes for the manufacture of synthetic gaseous fuels from coal or similar carbonaceous materials normally require the reaction of carbon with steam, alone or in combination with oxygen, at temperatures between about 1200°F (649°C) and about 2500°F (1371°C) to produce a gas which may contain some methane but consists primarily of hydrogen and carbon monoxide. This gas can be used directly as a synthesis gas or a fuel gas with little added processing or can be reacted with additional steam to increase the hydrogen-to-carbon monoxide ratio and then fed to a catalytic methanation unit for reaction of the carbon monoxide and the hydrogen to produce methane and water. It has been shown that processes of this type can be improved by carrying out the initial gasification step in the presence of a catalyst containing an alkali metal constituent. The alkali metal constituent accelerates the steam-carbon gasification reaction and thus permits the generation of synthesis gas at somewhat lower temperatures than would otherwise be required. Processes of this type are costly because of the large quantities of heat that must be supplied to sustain the highly endothermic steam-carbon reaction. One method of supplying this heat is to inject oxygen directly into the gasifier and burn a portion of the carbon in the feed material being gasified. This method is highly expensive in that it requires the existence of a plant to manufacture the oxygen. Other methods for supplying the heat have been suggested, but these, like that of injecting oxygen, are expensive.

It has been found that difficulties associated with processes of the type described above can largely be avoided by carrying out the reaction of steam with carbon in the presence of a carbon-alkali metal catalyst and substantially equilibrium quantities of added hydrogen and carbon monoxide. Laboratory work and pilot plant tests have shown that catalysts produced by the reaction of carbon and alkali metal compounds such as potassium carbonate to form carbon-alkali metal compounds or complexes will, under the proper reaction conditions, equilibrate the gas phase reactions occurring during gasification to produce methane and at the same time supply substantial amounts of exothermic heat within the gasifier. This additional exothermic heat of reaction essentially balances the overall endothermicity of the reactions involving solid carbon and thus results in a substantially thermoneutral process in which the injection of large amounts of oxygen or the use of other expensive methods of supplying heat are eliminated.

The catalytic effect of carbon-alkali metal catalysts on the gas phase reactions, as distinguished from the solid-gas reactions or the reactions of carbon with steam hydrogen, or carbon dioxide, allows the following exothermic reactions to contribute substantially to the presence of methane in the effluent gas and drastically reduces the endothermicity of the overall reaction:

(1) $2CO+2H_2 \rightarrow CO_2+CH_4$ (exothermic)
(2) $CO+3H_2 \rightarrow H_2O+CH_4$ (exothermic)
(3) $CO_2+4H_2 \rightarrow 2H_2O+CH_4$ (exothermic)

Under the proper operating conditions, these reactions can be made to take place within the gasification zone and supply large amounts of methane and additional exothermic heat which would otherwise have to be supplied by the injection of oxygen or other means. Laboratory and pilot plant tests have shown that constituents of the raw product gas thus produced are present in equilibrium concentrations at reaction conditions and consist primarily of hydrogen, carbon monoxide, carbon dioxide, methane, and steam. It has been proposed to utilize steam gasification in the presence of a carbon-alkali metal catalyst to produce a high Btu (calorific value) product gas by treating the raw product gas for removal of steam and acid gases, principally carbon dioxide and hydrogen sulfide; cryogenically separating carbon monoxide and hydrogen in amounts equivalent to their equilibrium concentration in the raw product gas from the methane in the treated gas; withdrawing methane as a high Btu product gas; and recycling the carbon monoxide and hydrogen to the gasifier. The presence in the gasifier of the carbon-alkali metal catalyst and equilibrium quantities of recycled carbon monoxide and hydrogen, which tend to suppress reactions that would otherwise produce additional hydrogen and carbon monoxide, results in a substantially thermoneutral reaction to produce essentially methane and carbon dioxide. Since the overall reaction is substantially thermoneutral, only a small heat input is required to preheat the carbonaceous feed material and to maintain the reactants at reaction temperatures by compensating for heat losses from the gasifier. This small amount of heat may be supplied by preheating the gaseous reactants in a conventional preheat furnace.

It has also been proposed to utilize steam gasification of a carbonaceous feed material in the presence of a carbon-alkali metal catalyst to produce an intermediate Btu (calorific value) product gas by treating the raw product gas withdrawn from the gasifier for the removal of the steam and acid gases, principally carbon dioxide and hydrogen sulfide; recovering a portion of the treated gas as the intermediate Btu (calorific value) product gas; contacting the remainder of the treated gas with steam in a steam reformer

under conditions such that the methane in the treated gas reacts with steam to produce additional hydrogen and carbon monoxide; and passing the effluent from the reformer into the gasifier. The amounts of hydrogen and carbon monoxide produced in the reformer compensate for the amounts of those gases removed in the treated gas that is withdrawn as intermediate Btu (calorific value) product gas. Thus the reformer effluent will normally contain carbon monoxide and hydrogen in amounts equivalent to the equilibrium quantities of those gases present in the raw product gas and will therefore supply the substantially equilibrium quantities of hydrogen and carbon monoxide required in the gasifier along with the carbon-alkali metal catalyst and steam to produce the thermoneutral reaction that results in the formation of essentially methane and carbon dioxide.

Although the above-described catalytic gasification processes result in the substantially thermoneutral reaction of steam with carbon to form a raw product gas containing equilibrium quantities of carbon monoxide, carbon dioxide, hydrogen, steam, and methane by recycling carbon monoxide and hydrogen in quantities equivalent to their concentration in the raw product gas to the gasifier and are therefore significant improvements over previous non-catalytic and catalytic processes, they do have several disadvantages. The equilibrium amount of methane in the raw product gas withdrawn from the gasifier is relatively low because it depends primarily on the gasifier temperature, the lower the amount of methane that is thermodynamically favored at equilibrium. In order to produce greater quantities of methane in the raw product gas withdrawn from the gasifier, it is therefore necessary to decrease the gasification temperature. If, however, the gasification temperature is decreased by a significant amount, the kinetics for the reaction of carbon with steam will be such that little if any gasification will occur. Normally, the catalytic gasification processes described above operate at a temperature of about 1300°F (704°C) and at a pressure of about 500 psig (3.45 KN/$m^2$). Under these conditions the raw product gas leaving the gasifier will contain approximately 23 mole percent methane, 23 mole percent hydrogen, 14 mole percent carbon dioxide, 7 mole percent carbon monoxide, and 31 mole percent unreacted steam. This raw product gas will normally be substantially free of vaporous or liquid constituents since all high molecular weight hydrocarbons are normally cracked under gasification conditions in the presence of the carbon-alkali metal catalyst. The failure to make greater quantities of methane and the inability to produce hydrocarbon liquids are distinct disadvantages inherent in both of these gasification processes. Furthermore, recycling the equilibrium amounts of carbon monoxide and hydrogen to the gasifier requires expensive equipment including cryogenic separators to remove the carbon monoxide and hydrogen from the methane in the raw product gas, and recycle compressors to increase the pressure of the recycle gas stream to a value high enough to allow its injection into the gasifier.

The present invention provides a catalytic process which at least in part alleviates the difficulties described above. In accordance with the invention, it has now been found that by integrating a catalytic devolatilization zone with a catalytic steam gasification zone, the concentration of methane in the raw product gas produced from a solid carbonaceous feed material can be substantially increased while decreasing the concentration of carbon monoxide and hydrogen, and, if desired, at the same time producing high-quality, hydrocarbon liquids. The carbonaceous feed solids are introduced into the devolatilization zone and contacted in the presence of a carbon-alkali metal catalyst with an added mixture of gases containing carbon monoxide and hydrogen at a temperature sufficiently high to devolatilize the carbonaceous feed solids but lower than conventional catalytic steam gasification temperatures, thereby producing char and a devolatilization effluent containing substantial amounts of methane. The char produced in the devolatilization zone is passed to a gasification zone where it is reacted with steam under gasification conditions in the presence of a carbon-alkali metal catalyst thereby producing a gaseous effluent containing substantially equilibrium quantities, at the gasification temperature and pressure, of carbon monoxide, carbon dioxide, hydrogen, methane and steam. This gaseous effluent is then introduced into the devolatilization zone where it is used as the added mixture of gases with which the carbonaceous feed solids are contacted. The devolatilization zone effluent will contain relatively high concentrations of methane and, depending on how the devolatilization zone is operated, may also contain vaporous constituents which can be condensed and recovered as high-quality hydrocarbon liquids. The devolatilization zone effluent is normally processed to recover a methane-containing gas ranging in quality from pure methane to an intermediate Btu (calorific value) gas containing a mixture of methane, carbon monoxide and hydrogen. If methane is the desired product, the carbon monoxide and hydrogen in the gas can be recycled to the gasification zone thereby supplying a portion of the heat required in the zone via the exothermic reaction of the carbon monoxide with the hydrogen to form methane. Any additional heat requirements can be provided by preheating the recycle stream of carbon monoxide and hydrogen or by injecting oxygen directly into the gasification zone to burn a portion of char. If an intermediate Btu (calorific value) product gas is desired, all of the heat requirements will normally be supplied by injecting oxygen into the gasfication zone. If air is used to supply the oxygen required in the gasification zone, the methane-containing product gas will be

diluted with nitrogen and will therefore possess a low Btu (calorific value) heating value.

The devolatilization zone is operated at a temperature of between 950°F (510°C) and 1150°F (621°C) and at a temperature of at least 100°F (55.5°C below) the temperature in the gasification zone and therefore equilibrium in the devolatilization zone will strongly favor the formation of methane by the reaction of the carbon monoxide with the hydrogen in the mixture of gases introduced into the zone. The carbon-alkali metal catalyst present in the devolatilization zone enables this exothermic methanation reaction to proceed at a reasonable rate even though the temperature in the zone is relatively low. The temperature in the zone, however, is maintained sufficiently high to devolatilize the carbonaceous feed solids thereby driving off gaseous hydrocarbons, including methane, in addition to vaporizing normally liquid products. If the devolatilization zone is operated such that the vaporized liquids have a relatively short residence time, substantial cracking of the vapors can be avoided and the effluent from the zone will contain vaporous constituents which can be condensed and recovered as liquid product. The hydrocarbon liquids produced by devolatilization in the presence of the carbon-alkali metal catalyst will have a lower oxygen content, a higher Btu (calorific value) content, and will be lighter and more stable than liquids obtained by conventional devolatilization processes carried out in the absence of a catalyst. The devolatilization zone effluent can be made to contain greater than equilibrium amounts of methane at the temperature and pressure in the zone by injecting the carbonaceous feed material near the top of the zone thereby sweeping the methane produced by devolatilization out of the zone before it has time to react with steam present to produce carbon monoxide and hydrogen.

The heat required in the devolatilization zone is supplied by the sensible heat in the gasification zone effluent, which is passed into the devolatilization zone, and the heat given off by the reaction of the added carbon monoxide with the added hydrogen to produce additional methane in the presence of the carbon-alkali metal catalyst at the relatively low temperature in the devolatilization zone. If the temperature in the devolatilization zone tends to become too high, it can be lowered by passing only a portion of the gasification zone effluent into the devolatilization zone. Normally, the only source of gaseous feed to the devolatilization zone will be the effluent from the gasification zone. In some cases it may be desirable to operate the devolatilization zone at a temperature sufficiently low to prevent any substantial reaction of steam with carbon in the carbonaceous feed solids. By utilizing the lower temperature devolatilization zone to catalytically produce methane, it is not necessary to operate the gasification zone at a relatively low gasification temperature which thermodynamically favors the production of methane and thus the gasification temperature can be raised to increase the reaction rate of steam with carbon in the char fed to the gasification zone thereby decreasing the size requirements of the reactor and significantly reducing the initial investment costs of the process.

In this process the carbon-alkali metal catalyst used in the devolatilization zone is prepared by impregnating said carbonaceous feed solids with an alkali metal compound-containing solution in a zone external to said devolatilization zone and to said gasification zone and thereafter heating the impregnated solids in the devolatilization zone which heating may be the contacting of carbonaceous feed solids with the mixture of gases in the presence of the catalyst at the temperature sufficiently high to devolatilize the carbonaceous feed solids and produce char and the devolatilization effluent.

The process of the invention provides an integrated devolatilization and steam gasification process carried out in the presence of a carbon-alkali metal catalyst which results in an increased production of methane over that obtainable by simply gasifying a carbonaceous feed material with steam in the presence of a carbon-alkali metal catalyst; and, if desired, the simultaneous production of high-quality, hydrocarbon liquids. By passing the gasification zone effluent into a devolatilization zone and operating that zone at a lower temperature than the gasification zone, carbon monoxide and hydrogen in the gasification zone effluent react to form methane thereby increasing the methane yield of the process and decreasing the amount of carbon monoxide and hydrogen that must be recycled to the gasification zone. The use of the low temperature devolatilization zone to promote the catalytic reaction of carbon monoxide with hydrogen to form methane allows the temperature in the gasification zone to be increased thereby increasing the reaction kinetics of steam with carbon in the gasification zone and allowing a reduction in the size of the gasification vessel. Thus, the process offers many advantages over catalytic gasification alone.

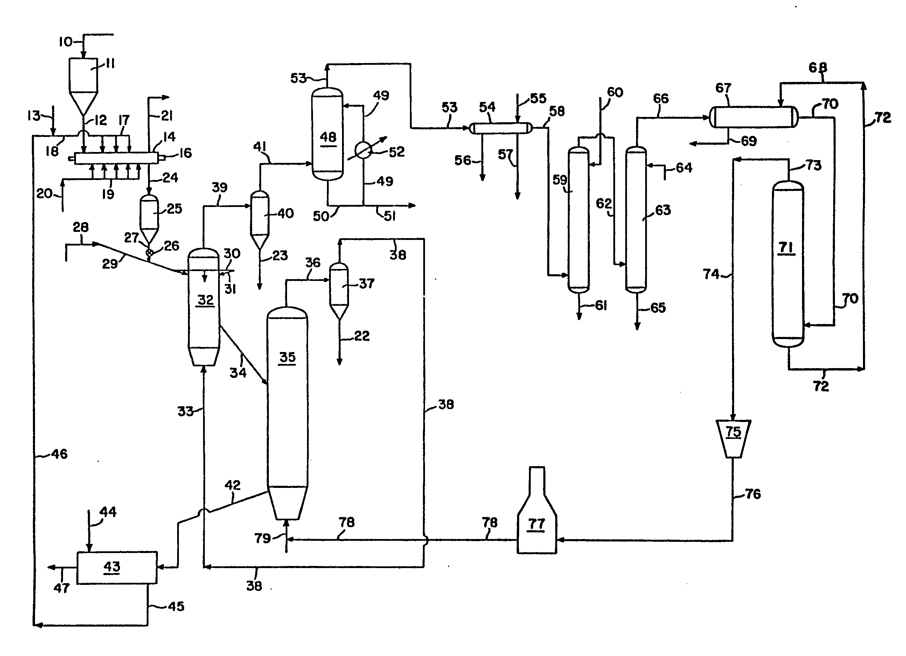

The invention is described with reference to the drawing which is a schematic flow diagram of an integrated catalytic devolatilization and steam gasification process.

The process depicted in the drawing is one for the production of methane by the devolatilization of bituminous coal, subbituminous coal, lignite, coal char, coke, coal liquefaction bottoms, oil shale, or similar carbonaceous solids in the presence of a carbon-alkali metal catalyst prepared by impregnating the feed solids with a solution of an alkali metal compound, a mixture of such compounds or a mixture of an alkali metal compound and some other metal compound, and thereafter heating the impregnated material to a temperature sufficient to produce an interaction between the alkali metal and the carbon present. The char produced by the devolatilization is then gasified with steam in the presence of a carbon-alkali metal catalyst and the resultant effluent gases are used as the gaseous medium in which the devolatilization is carried out.

The solid feed material that has been crushed to a particle size of about 8 mesh (2.38 mm sieve opening) or smaller on the U.S. Sieve Series Scale is passed into line 10 from a feed preparation plant or storage facility that is not shown in the drawing. The solids introduced into line 10 are fed into a hopper or similar vessel 11 from which they are passed through line 12 into feed preparation zone 14. This zone contains a screw conveyor or similar device, not shown in the drawing, that is powered by motor 16, a series of spray nozzles or similar devices 17, for the spraying of an alkali metal-containing solution supplied through line 18 on to the solids as they are moved through the preparation zone by the conveyor, and a similar set of nozzles or the like 19 for the introduction of a hot dry gas, such as flue gas, into the preparation zone. The hot gas, supplied through line 20, serves to heat the impregnated solids and drive off the moisture. A mixture of water vapor and gas is withdrawn from zone 14 through line 21 and passed to a condenser, not shown, from which water may be recovered for use as make-up or the like. The majority of the alkali metal-containing solution is recycled through line 46 from the alkali metal recovery portion of the process, which is described hereafter. Any make-up alkali metal solution required may be introduced into line 18 via line 13.

It is preferred that sufficient alkali metal-containing solution be introduced into preparation zone 14 to provide from 1 to 50 weight percent of an alkali metal compound, a mixture of such compounds or a mixture of an alkali metal compound and some other metal compound on the coal or other carbonaceous solids. From 5 to 30 weight percent is generally adequate. The dried impregnated solid particles prepared in zone 14 are withdrawn through line 24 and passed to a closed hopper or similar vessel 25 from which they are discharged through a star wheel feeder or equivalent device 26 in line 27 at an elevated pressure sufficient to permit their entrainment into a stream of high pressure steam, recycle process gas, inert gas, or other carrier gas introduced into line 29 via line 28. The carrier gas and entrained solids are passed through line 29 into manifold 30 and fed from the manifold through feed lines 31 and nozzles, not shown in the drawing, into devolatilizer 32. In lieu of or in addition to hopper 25 and star wheel feeder 26, the feed system may employ parallel lock hoppers, pressurised hoppers, aerated standpipes operated in series, or other apparatus to raise the input feed solid stream to the required pressure level.

Devolatilizer 32 contains a fluidized bed of carbonaceous solids extending upward within the vessel above an internal grid or similar distribution device not shown in the drawing. The bed is maintained in the fluidised state by means of steam, hydrogen, carbon monoxide, carbon dioxide and methane introduced into the bottom of the devolatilizer through bottom inlet line 33. These gases may be introduced into the devolatilizer through multiple nozzles to obtain a uniform distribution of the injected fluid and reduce the possibility of channeling and related problems. It is generally preferred to operate the fluidized bed devolatilizer at a pressure between 100 ($6.9\times10^2$ $KN/m^2$) and 1500 psig ($1.03\times10^4$ $KN/m^2$) the most preferred range of operation being between 200 ($1.38\times10^3$ $KN/m^2$) and 800 psig ($5.5\times10^3$ $KN/m^2$).

Within the fluidized bed in devolatilizer 32, the carbonaceous solids impregnated with the alkali metal compound or compounds in the alkali metal-containing solution introduced into feed preparation zone 14 are subjected to a temperature within the range between 850°F (454°C) and 1300°F (704°C) preferably between 950°F (510°C) and 1150°F (621°C). At such temperature the alkali metal constituents interact with the carbon in the carbonaceous solids to form a carbon-alkali metal catalyst and the carbonaceous solids simultaneously undergo devolatilization whereby methane, ethane, and other gaseous constituents are driven off the solids and compounds that are normally liquids at ambient conditions are vaporized. In the presence of the carbon-alkali metal catalyst, the carbon monoxide and hydrogen in the fluidizing gases injected into the devolatilizer through bottom inlet line 33 react exothermically to form methane. The liberated exothermic heat together with the sensible heat in the fluidizing gases serve to supply the heat required to effect devolatilization of the carbonaceous feed solids. Devolatilization in the presence of the carbon-alkali metal catalyst produces light liquids substantially free of tars and having a higher Btu content and lower oxygen content than liquids obtained from conventional devolatilization processes.

The char produced during devolatilization in vessel 32 contains carbon-alkali metal catalyst and is withdrawn from the fluidized bed through transfer line 34, passed through a valve, not shown in the drawing, and injected into a fluidized bed of carbonaceous solids extending upward within gasifier 35 above an internal grid or similar distribution device not shown in the drawing. The char solids are maintained in a fluidized state within the gasifier by means of steam, carbon monoxide, and hydrogen injected into the gasifier through bottom inlet 79. Normally, the gasifier pressure will range between 100 psig ($6.9\times10^2$ $KN/m^2$) and 1500 psig ($1.03\times10^4$ $KN/m^2$), preferably between 200 psig ($1.38\times10^3$ $KN/m^2$) and 800 psig ($5.5\times10^3$ $KN/m^2$). The temperature in the gasifier is normally maintained at least about 100°F (55.5°C) above the temperature in the devolatilizer and will preferably range between 200°F (392°C) and 400°F (752°C) above the devolatilizer temperature. Normally, the gasifier temperature will range between 1100°F (593°C) and 1500°F (816°C), preferably between 1300°F (704°C) and 1500°F (816°C).

Under the conditions in the gasifier, the steam injected reacts with carbon in the char to produce a gas composed of hydrogen, carbon monoxide, carbon dioxide, and methane. By carrying out the gasification process in the presence of a carbon-alkali metal catalyst, the gas phase reactions occurring during gasification are equilibrated. Substantial quantities of exothermic heat are released as a result of the reaction of hydrogen with carbon oxides and the reaction of carbon monoxide with steam in the presence of the carbon-alkali metal catalyst. This exothermic heat helps to balance the endothermic heat consumed by the reaction of steam with carbon, thereby reducing the amount of heat that ordinarily needs to be supplied to the gasifier.

The carbon-alkali metal catalyst utilized in the process of the invention is prepared by impregnating the carbonaceous feed material with an alkali metal-containing solution and then subjecting the impregnated solids to a temperature above about 800°F (427°C) in the devolatilizer. The carbon-alkali metal catalyst is then transferred along with the char produced in the devolatilizer into the gasifier. It will be understood that the carbon-alkali metal catalyst utilized in the process of this invention can be prepared without impregnation onto the carbonaceous solids to be devolatilized and then gasified, and without heating in the devolatilizer or gasifier. The heating step, for example, may be carried out in a solid feed preparation zone or in an external heater. The carbonaceous solids used will in most instances be the ones that are to be devolatilized and then gasified but in some variations of the process carbonaceous materials other than the feed solids may be used. In some cases inert carriers having carbon deposited on their outer surface may be used. Suitable inert carriers include silica, alumina, silica-alumina, zeolites, and the like. The catalyst particles, whether composed substantially of carbon and an alkali metal constituent or made up of carbon and an alkali metal constituent deposited on an inert carrier, may range from fine powders to coarse lumps, particles between 4 and 100 mesh on the U.S. Sieve Series Scale generally being preferred (between 4.76 mm and 0.149 mm sieve opening).

Any of a variety of alkali metal constituents can be used in preparing the carbon-alkali metal catalyst. Suitable constituents include the alkali metals themselves and alkali metal compounds such as alkali metal carbonates, bicarbonates, formates, oxalates, hydroxides, acetates, sulfides, nitrates, and mixtures of these and other similar compounds. All these are not equally effective and hence a catalyst prepared from certain alkali metal constituents can be expected to give somewhat better results under certain conditions than do others. In general, cesium, potassium, sodium and lithium salts derived from organic or inorganic acids having ionization constants less than about $1\times10^{-3}$ and alkali metal hydroxides are preferred. The cesium compounds are the most effective, followed by the potassium, sodium and lithium compounds in that order. Because of their high activity, relatively low cost compared to cesium compounds, and ready availability, potassium compounds or sodium compounds are generally employed. Potassium carbonate and potassium hydroxide are especially effective.

In the embodiment of the invention shown in the drawing, the alkali metal constituent or constituents and the carbonaceous solids are combined to form an intimate mixture by dissolving a water-soluble alkali metal compound, a mixture of such compounds, or a mixture of a water-soluble alkali metal compound and some other metal compound in an aqueous carrier, impregnating the carbonaceous solids with the resulting aqueous solution by soaking or spraying the solution onto the particles and thereafter drying the solids. It will be understood that other methods of forming such an intimate mixture may be used. For example, in some cases the carbonaceous material can be impregnated by suspending a finely divided alkali metal or alkali metal compound in a hydrocarbon solvent or other inert liquid carrier of suitably low viscosity and high volatility and thereafter treating the solids with the liquid containing the alkali metal constituent. In other cases it may be advantageous to pelletize a very finely divided alkali metal or alkali metal compound with carbon in an oil or similar binder and then heat the pellets to an elevated temperature. Other catalyst preparation methods, including simply mixing finely divided carbonaceous material with a powdered alkali metal salt and thereafter heating the mixture to the desired temperature, can in some cases, be used.

The mechanisms which take place as the result of combining the carbonaceous solids and alkali metal constituents and then heating them to elevated temperatures are not fully understood. Apparently, the alkali metal reacts with the carbon to form carbon-alkali metal compounds and complexes. Studies have shown that neither carbonaceous solids nor the alkali metal constituents alone are fully effective for establishing equilibrium conditions for gas phase reactions involving steam, hydrogen, carbon monoxide carbon dioxide, and methane and that catalytic activity is obtained only when a compound or complex of the carbon and alkali metal is present in the system. Both constituents of the catalyst are therefore necessary. Experience has shown that these catalysts are resistant to degradation in the presence of sulfur compounds, that they resist sintering at high temperatures, and that they bring gas phase reactions involving gases normally produced during coal gasification and devolatilization into equilibrium. As a result of these and other beneficial properties, these catalysts have pronounced advantages over other catalysts employed in the past.

Referring again to the drawing, the gas leaving the fluidized bed in gasifier 35 passes through the upper section of the gasifier, which serves as a disengagement zone where particles too heavy to be entrained by the gas leaving the vessel are returned to the bed. If desired, this disengagement zone may include one or more cyclone separators or the like for removing relatively large particles from the gas. The gas withdrawn from the upper part of the gasifier through line 36 will normally contain an equilibrium mixture at reaction temperature and pressure of methane, carbon dioxide, hydrogen, carbon monoxide and unreacted steam. Also present in this gas are hydrogen sulfide, ammonia and other contaminants formed from the sulfur and nitrogen contained in the feed material and entrained fines. This raw product gas is introduced into cyclone separator or similar device 37 where the fine particles are removed. The gas from which the solids have been separated is withdrawn overhead from separator 37 and passed through lines 38 and 33 into devolatilizer 32 where it serves as the fluidizing medium for the bed of carbonaceous solids and also gives up a portion of its sensible heat to provide a portion of the heat required for devolatilization. The fine particles removed from the gas in separator 37 are discharged downward through dip leg 22 and may be returned to the gasifier or passed to the alkali metal recovery portion of the process.

The integration of the devolatilizer with the gasifier and the carrying out of both devolatilization and gasification in the presence of a carbon-alkali metal catalyst as described above has been found to have many advantages. Since the devolatilizer is operated at a temperature substantially lower than the gasification temperature, thermodynamics favor the formation of more methane in the devolatilizer and allow the integrated process to produce a raw product gas containing greater amounts of methane than could otherwise be produced at the higher gasification temperature where thermodynamics favor the formation of less amounts of methane. The carbon-alkali metal catalyst in the devolatilizer catalyzes the reaction of the carbon monoxide and hydrogen in the gasifier effluent, which is passed into the devolatilizer, thereby producing additional amounts of methane and providing exothermic heat for devolatilization. By injecting the carbonaceous feed solids at a position relatively high in the fluidized bed devolatilizer, methane produced by devolatilization, rather than by the reaction of hydrogen with carbon monoxide, will be carried out of the devolatilizer before it can react with steam to form hydrogen and carbon monoxide and the devolatilizer effluent will therefore contain greater than equilibrium amounts of methane at the temperature and pressure conditions in the devolatilizer. Furthermore, injection of the solids at a point near the top of the fluidized bed will result in the production of high quality, light, hydrocarbon liquids since the vapors produced by devolatilization will have a relatively short residence time in the devolatilizer and will therefore not be cracked into gases. If the exothermic heat produced by the methanation reactions occurring in the devolatilizer plus the sensible heat in the gasifier effluent provide more heat than is required in the devolatilizer to preheat the carbonaceous feed material and carry out the devolatilization reactions, a portion of the gasifier product gas may be by-passed around the devolatilizer. If there is not enough heat in the devolatilizer or if the carbon-alkali metal catalyst which is produced in the devolatilizer is not of high enough activity and needs to be exposed to higher temperatures to increase activity, then char containing carbon-alkali metal catalyst formed in the gasifier can be passed back into the devolatilizer after it has been exposed to the higher gasification temperature.

It will be understood that although the devolatilizer is described as a fluidized bed reactor it may consist of an entrained flow transfer line, a free fall zone, a fixed bed reactor or the like. If it is desired to produce a raw product gas from the devolatilization zone without producing any liquids, the carbonaceous feed may be injected toward the bottom of the fluidized bed rather than near the top as described above thereby allowing time for vaporized liquids to crack and form low molecular weight gaseous constituents. The temperature in the devolatilizer will normally be maintained as low as possible to obtain maximum methane formation but high enough to produce substantial devolatilization. In some cases it may be desirable to maintain the temperature in the devolatilizer sufficiently below the gasifier temperature to prevent any substantial reaction of steam with the carbonaceous feed solids in the devolatilizer.

Referring again to the drawing, the gas leaving the fluidized bed in devolatilizer 32 is withdrawn from the upper part of the devolatilizer through line 39 and will normally contain vaporous constituents produced by the devolatilization of the carbonaceous feed material, and gaseous constituents including methane, carbon dioxide, hydrogen, carbon monoxide and unreacted steam. Hydrogen sulfide, ammonia and other contaminants including fine particulates will also be present in the devolatilizer effluent gas. This raw product gas, which will contain more methane than the effluent withdrawn overhead from gasifier 35, is introduced into cyclone separator or similar device 40 where the particulates are removed. The gaseous and vaporous constituents from which the solids have been separated are taken overhead from separator 40 through line 41 and the particulates are discharged downward through dip leg 23. These fine solids are normally passed to the bottom of the fluidized bed in gasifier 35.

The particulate-free devolatilizer effluent withdrawn overhead from separator 40 is passed through line 41 into the bottom of hot oil scrubber or similar device 48. Here the gaseous and

vaporous constituents pass upward through the contacting zone in the scrubber where they come in direct contact with a downflowing stream of cool oil introduced into the top of the scrubber through line 49. As the gaseous and vaporous constituents rise through the contacting zone, heat from the vaporous constituents is absorbed by the cool oil thereby causing the vapors to condense forming liquid hydrocarbons which are withdrawn from the bottom of the scrubber through line 50. A portion of these liquids is recycled through heat exchanger 52 where they are cooled to produce the low temperature oil introduced into the top of the hot oil scrubber through line 49. The remainder of the liquids are withdrawn through line 51 as hydrocarbon liquid product. It has been found that these liquids, which are produced by devolatilization in the presence of a carbon-alkali metal catalyst, are substantially free of tars, have a low oxygen content, a high Btu (calorific value) content, a high stability and are generally higher quality liquids than can ordinarily be obtained by conventional devolatilization processes carried out in the absence of a catalyst. These high quality liquids are suitable for use as fuel, solvents, refinery and petrochemical feedstocks, and the like.

The devolatilizer effluent gas from which the vaporous constituents have been condensed is withdrawn overhead from hot oil scrubber 48 through line 53 and passed to waste heat boiler 54 where it is further cooled by indirect heat exchange with water introduced through line 55. Sufficient heat is transferred from the gas to the water to convert it into stream which is withdrawn through line 56. During this cooling step, unreacted steam in the gas is condensed out and withdrawn as condensate through line 57. The cool gas leaving waste heat boiler 54 through line 58 is passed to water scrubber 59. Here the gas stream passes upward through the scrubber where it comes in contact with water injected into the top of the scrubber through line 60. The water absorbs ammonia and a portion of the hydrogen sulfide in the gas stream and is withdrawn from the bottom of the scrubber through line 61 and passed to downstream units for further processing. The water-scrubbed gas stream is withdrawn from the scrubber through line 62 and is now ready for treatment to remove bulk amounts of hydrogen sulfide and other acid gases.

The gas stream is passed from water scrubber 59 through line 62 into the bottom of solvent scrubber 63. Here the gas passes upward through the contacting zone in the scrubber where it comes in contact with a downflowing stream of solvent such as monoethanolamine, diethanolamine, a solution of sodium salts of amino acids, methanol, hot potassium carbonate or the like introduced into the upper part of the solvent scrubber through line 64. If desired, the solvent scrubber may be provided with spray nozzles, perforated plates, bubble cap plates, packing or other means for promoting intimate contact between the gas and the solvent. As the gas rises through the contacting zone, hydrogen sulfide, carbon dioxide and other acid gases are absorbed by the solvent, which exits the scrubber through line 65. The spent solvent containing carbon dioxide, hydrogen sulfide and other contaminants is passed through line 65 to a stripper, not shown in the drawing, where it is contacted with steam or other stripping gas to remove the absorbed contaminants and thereby regenerate the solvent. The regenerated solvent may then be reused by injecting it back into the top of the scrubber via line 64.

A clean intermediate Btu (calorific value) gas containing essentially methane, hydrogen, and carbon monoxide is withdrawn overhead from the solvent scrubber via line 66. In one embodiment of the invention, this intermediate Btu (calorific value) gas can be withdrawn from the process as a methane-containing product gas and used as a fuel to supply the heat requirement of other industrial plants or for other purposes. In the preferred embodiment of the invention, which is depicted in the drawing, this gas stream is further processed to remove the carbon monoxide and hydrogen thereby producing a high Btu (calorific value) product gas consisting essentially of methane, and the recovered carbon monoxide and hydrogen are recycled to the gasifier where they react exothermically in the presence of the carbon-alkali metal catalyst to produce methane thereby supplying a portion of the heat required to operate the gasifier. If the intermediate Btu (calorific value) gas is removed as product, the heat requirements of the gasifier will normally be supplied by injecting oxygen directly into the gasifier where it reacts exothermically with a portion of the carbon in the char. In some cases, it may be desirable to utilize air as the source of the oxygen. If air is used, th clean gas removed overhead from solvent scrubber 63 will contain substantial amounts of nitrogen and will therefore normally be a low Btu (calorific value) gas.

Referring again to the drawing, the intermediate Btu (calorific value) gas withdrawn overhead from solvent scrubber 63 through line 66 is introduced into heat transfer unit 67 where it passes in indirect heat exchange with liquid methane introduced through line 68. The methane vaporizes within the heat transfer unit and is discharged as methane product and gas through line 69. The vaporizing methane chills the intermediate Btu (calorific value) gas, which is primarily composed of methane, hydrogen, and carbon monoxide, to a low temperature approaching that required for liquefaction of the methane contained in the gas, after which the chilled gas is passed through line 70 into cryogenic unit 71. Here the gas is further cooled by conventional means until the temperature reaches a value sufficiently low to liquefy the methane under the pressure conditions existing in the unit. Compressors and other auxiliaries associated with the cryogenic unit are not shown.

The amount of pressure required for the liquefaction step will depend in part upon the pressure losses which are incurred in the various portions of the system. A substantially pure stream of liquified methane is removed from the bottom of the cryogenic unit via line 72 and passed through line 68 into heat transfer unit 67 as described earlier. Hydrogen and carbon monoxide are withdrawn overhead from cryogenic unit 71 through line 73. Normally, the cryogenic unit is operated and designed in such a manner that less than about 10 mole percent methane, preferably less than about 5 mole percent remains in the hydrogen and carbon monoxide stream removed through line 73.

The hydrogen and carbon monoxide removed from cryogenic unit 71 through line 73 is passed through line 74 to compressor 75 where its pressure is increased to a value from 25 psi ($1.72\times10^2$ $KN/m^2$) to 150 psi ($10.3\times10^2$ $KN/m^2$) above the operating pressure in gasifier 35. The pressurized gas is withdrawn from compressor 75 through line 76 and passed to preheat furnace or similar device 77 where the carbon monoxide and hydrogen are preheated. The preheated mixture is removed from furnace 77, passed through line 78 and mixed with steam in line 79. The resultant mixture of steam, carbon monoxide, and hydrogen is then injected into gasifier 35.

As pointed out previously, substantial quantities of exothermic heat are released in the gasifier as a result of the reaction of hydrogen with carbon oxides and the reaction of carbon monoxide with steam. Thus, the carbon monoxide and hydrogen in the preheat furnace effluent comprise a substantial portion of the heat input into the gasifier. If the amounts of carbon monoxide and hydrogen injected into the gasifier were equivalent to the amounts withdrawn from the gasifier through line 36, the exothermic heat released in the gasifier would substantially balance the heat requirements of the gasifier. However, a portion of the carbon monoxide and hydrogen in the gasifier effluent react in devolatilizer 32 to produce additional methane and therefore the amounts of these gases available for reinjection into the gasifier are less than the amounts withdrawn from the gasifier. To compensate for the loss of exothermic heat in the gasifier caused by the reaction of carbon monoxide and hydrogen in the devolatilizer, the preheat furnace must be operated at a higher temperature or oxygen must be injected into the gasifier to generate heat by burning a portion of the char. This extra expense may be more than offset by the lesser amounts of carbon monoxide and hydrogen that must be recycled to the gasifier which in turn reduces the equipment costs required for both the compressor and the preheat furnace.

In the system shown in the drawing, a stream of high ash content char particles is withdrawn through line 42 from gasifier 35 in order to control the ash content of the system and permit the recovery and recycle of alkali metal constituents of the catalyst. The solids in line 42, which may be combined with fines recovered from the gasifier overhead gas through dip leg 22, are passed to alkali metal recovery unit 43. The recovery unit will normally comprise a multistage countercurrent leaching system in which the high ash content particles containing alkali metal constituents are countercurrently contacted with water introduced through line 44. The first stage of the catalyst recovery unit may utilize calcium hydroxide digestion to convert water-insoluble catalyst constituents into water-soluble constituents. Such a digestion step is described in detail in U.S. Patent No. 4,159,195. An aqueous solution of alkali metal compounds is withdrawn from the unit through line 45 and recycled through lines 46 and 18 to feed preparation zone 14. Ash residues from which soluble alkali metal compounds have been leached are withdrawn from the recovery unit through line 47 and may be disposed of as landfill.

It will be apparent from the foregoing that the invention provides an integrated coal devolatilization and steam gasification process in which both the devolatilization and gasification steps are carried out in the presence of a carbon-alkali metal catalyst to produce a raw product gas containing a much higher concentration of methane than can be obtained in conventional gasification processes. The process of the invention can also be operated in such a manner as to produce high-quality, hydrocarbon liquids concurrently with the methane-containing product gas.

**Claims**

1. An integrated catalytic devolatilization and steam gasification process for the production of a methane-containing gas from carbonaceous feed solids which comprises:

(a) contacting said carbonaceous feed solids in a devolatilization zone with a mixture of gases containing carbon monoxide and hydrogen in the presence of a carbon-alkali metal catalyst at a temperature sufficiently high to devolatilize said carbonaceous feed solids and produce char and a devolatilization effluent;

(b) recovering a methane-containing gas as product from said devolatilization zone effluent;

(c) passing said char produced in said devolatilization zone into a gasification zone;

(d) reacting said char with steam in said gasification zone under gasification conditions in the presence of a carbon-alkali metal catalyst;

(e) withdrawing from said gasification zone an effluent gas containing carbon monoxide, carbon dioxide, hydrogen, methane and steam;

(f) passing said effluent gas withdrawn from said gasification zone to said devolatilization zone thereby supplying the mixture of gases with which said carbonaceous feed solids are contacted in said devolatilization zone in step (a); and

(g) operating said devolatilization zone at a

temperature of between 950°F and 1150°F (510°C and 621°C) and at a temperature of at least 100°F (55.5°C) below the temperature in said gasification zone, heat required in said devolatilization zone being supplied by the sensible heat in the effluent from said gasification zone and by the heat given off by the formation of methane by the exothermic reaction of the carbon monoxide with the hydrogen in said mixture of gases in said devolatilization zone; characterised in that the carbon-alkali metal catalyst used in the devolatilization zone is prepared by impregnating said carbonaceous feed solids with an alkali metal compound-containing solution in a zone external to said devolatilization zone and to said gasification zone and thereafter heating the impregnated solids in the devolatilization zone which heating may be the contacting of carbonaceous feed solids with the mixture of gases in the presence of the catalyst at a high temperature as defined in step (a).

2. A process according to claim 1 in which the carbonaceous feed solids are introduced sufficiently high into said devolatilization zone so that said devolatilization zone effluent contains both gaseous constituents and substantially tar-free vaporous constituents, and in which said substantially tar-free vaporous constituents are condensed to recover substantially tar-free hydrocarbon liquids, methane is recovered from the gaseous constituents and hydrogen and carbon monoxide contained in said gaseous constituents are recycled to said gasification zone.

3. A process according to either of Claims 1 and 2 in which substantially all of said effluent gas withdrawn from said gasification zone is used as said mixture of gases introduced into said devolatilization zone.

4. A process according to any one of Claims 1 to 3 in which said devolatilization zone is a fluidised bed devolatilization zone.

**Patentansprüche**

1. Integriertes katalytisches Devolatilisierungs- und Dampfvergasungsverfahren für die Herstellung von methanhaltigem Gas aus kohlenstoffhaltigen Einsatzmaterialfeststoffen, bei dem

(a) die kohlenstoffhaltigen Einsatzmaterialfeststoffe in einer Devolatilisierungszone mit einer Mischung von Gasen, die Kohlenmonoxid und Wasserstoff enthält, in Gegenwart eines Kohlenstoff-Alkalimetallkatalysators bei einer ausreichend hohen Temperatur kontaktiert werden, um die kohlenstoffhaltigen Einsatzmaterialfeststoffe zu devolatilisieren und Holzkohle und ein austretendes Devolatilisierungsprodukt zu erzeugen,

(b) ein methanhaltiges Gas als Produkt aus dem aus der Devolatilisierungszone austretenden Produkt gewonnen wird,

(c) die in der Devolatilisierungszone erzeugte Holzkohle in eine Vergasungszone geleitet wird,

(d) die Holzkohle in der Vergasungszone unter Vergasungsbedingungen in Gegenwart eines Kohlenstoff-Alkalimetallkatalysators mit Dampf zur Umsetzung gebracht wird,

(e) aus der Vergasungszone ein austretendes Gas abgezogen wird, das Kohlenmonoxid, Kohlendioxid, Wasserstoff, Methan und Dampf enthält,

(f) das aus der Vergasungszone abgenommene, austretende Gas zu der Devolatilisierungszone geleitet wird und dadurch die Mischung von Gasen liefert, mit der die kohlenstoffhaltigen Einsatzmaterialfeststoffe in der Devolatilisierungszone in Stufe (a) kontaktiert werden und

(g) die Devolatilisierungszone bei einer Temperatur von 510 bis 621°C und bei einer Temperatur von mindestens 55.5°C unterhalb der Temperatur in der Vergasungszone betrieben wird, wobei die in der Devolatilisierungszone erforderliche Wärme von der Eigenwärme des Austrittsgases aus der Vergasungszone und durch die bei der Bildung von Methan durch die exotherme Reaktion von Kohlenmonoxid mit Wasserstoff in der Mischung von Gasen in der Devolatilisierungszone abgegebenen Wärme geliefert wird, dadurch gekennzeichnet, daß der Kohlenstoff-Alkalimetallkatalysator, der in der Devolatilisierungszone verwendet wird, hergestellt wird, indem die kohlenstoffhaltigen Einsatzmaterialfeststoffe mit einer eine Alkalimetallverbindung enthaltenden Lösung in einer sich außerhalb von der Devolatilisierungszone und der Vergasungszone befindenden Zone imprägniert werden und danach die imprägnierten Feststoffe in der Devolatilisierungszone erhitzt werden, wobei das Erhitzen das Kontaktieren der kohlenstoffhaltigen Einsatzmaterialfeststoffe mit der Mischung der Gase in Gegenwart des Katalysators bei einer hohen Temperatur, wie in Stufe (a) definiert, sein kann.

2. Verfahren nach Anspruch 1, dadurch gekennzeichnet, daß die kohlenstoffhaltigen Einsatzmaterialfeststoffe ausreichend hoch in die Devolatilisierungszone eingeführt werden, so daß das aus der Devolatilisierungszone austretende Gas sowohl gasförmige Bestandteile als auch im wesentlichen teerfreie dampfförmige Bestandteile enthält, und daß die im wesentlichen teerfreien dampfförmigen Bestandteile kondensiert werden, um im wesentlichen teerfreie Kohlenwasserstofflüssigkeiten zu gewinnen, Methan aus den gasförmigen Bestandteilen gewonnen wird und Wasserstoff und Kohlenmonoxid, die in den gasförmigen Bestandteilen enthalten sind, zu der Vergasungszone zurückgeführt werden.

3. Verfahren nach Anspruch 1 oder 2, dadurch gekennzeichnet, daß im wesentlichen das gesamte von der Vergasungszone abgezogene, austretende Gas als die Mischung von Gasen verwendet wird, die in die Devolatilisierungszone eingeleitet wird.

4. Verfahren nach einem der Ansprüche 1 bis 3, dadurch gekennzeichnet, daß die Devolatilisierungszone eine Wirbelbett-Devolatilisierungszone ist.

**Revendications**

1. Procédé catalytique intégré de dévolatilisation et de gazéification par la vapeur d'eau pour la production d'un gaz contenant du méthane à partir de charges solides carbonées, qui comprend les étapes suivantes:

(a) mettre en contact ces charges solides carbonées dans une zone de dévolatilisation avec un mélange de gaz contenant de l'oxyde de carbone et de l'hydrogène en présence d'un catalyseur carbone-métal alcalin, à une température suffisamment élevée pour dévolatiliser les charges solides carbonées et pour produire un produit de carbonisation et un effluent de dévolatilisation;

(b) récupérer comme produit, à partir de l'effluent de la zone de dévolatilisation, un gaz contenant du méthane;

(c) introduire le produit de carbonisation, obtenu dans la zone de dévolatilisation, dans une zone de gazéification;

(d) faire réagir le produit de carbonisation avec de la vapeur d'eau dans la zone de gazéification dans des conditions de gazéification en présence d'un catalyseur carbonemétal alcalin;

(e) retirer de la zone de gazéification un gaz effluent contenant de l'oxyde de carbone, de l'anhydride carbonique, de l'hydrogène, du méthane et de la vapeur d'eau;

(f) introduire le gaz effluent retiré de la zone de gazéification dans la zone de dévolatilisation, ce qui fournit le mélange de gaz avec lequel les solides d'alimentation carbonés sont mis en contact dans la zone de dévolatilisation dans l'étape (a); et

(g) faire fonctionner la zone de dévolatilisation à une température comprise entre 950°F et 1150°F (510°C et 621°C) et à une température inférieure d'au moins 100°F (55.5°C) à la température de la zone de gazéification, la chaleur nécessitée dans la zone de dévolatilisation étant fournie par la chaleur sensible de l'effluent provenant de la zone de gazéification et par la chaleur dégagée par la formation de méthane par la réaction exothermique de l'oxyde de carbone avec l'hydrogène dans le mélange de gaz dans la zone de dévolatilisation; charactérisé en ce que le catalyseur carbone-métal alcalin employé dans la zone de dévolatilisation est préparé par imprégnation des charges solides carbonées avec une solution contenant un composé de métal alcalin, dans une zone extérieure à la zone de dévolatilisation et à la zone de gazéification et ensuite chauffage des solides imprégnés dans la zone de dévolatilisation, ce chauffage pouvant être la mise en contact des charges solides carbonées avec le mélange de gaz en présence du catalyseur à une température élevée telle qu'elle est définie dans l'étape (a).

2. Procédé selon la revendication 1, caractérisé en ce que les charges solides carbonées sont introduits suffisamment haut dans la zone de dévolatilisation pour que l'effluent de la zone de dévolatilisation contienne à la fois des constituants gazeux et des constituants en phase vapeur pratiquement exempts de goudrons, et en ce que les constituants en phase vapeur pratiquement exempts de goudrons sont condensés pour récuper des liquides hydrocarbonés pratiquement exempts de goudrons, en ce que le méthane est récupéré à partir des constituents gazeux et en ce que l'hydrogène et l'oxyde de carbone contenus dans les constituants gazeux sont recyclés à la zone de gazéification.

3. Procédé selon la revendication 1 ou 2, caractérisé en ce que pratiquement la totalité du gaz effluent retiré de la zone de gazéification est employée comme mélange de gaz introduit dans la zone de dévolatilisation.

4. Procédé selon l'une quelconque des revendications 1 à 3, caractérisé en ce que la zone de dévolatilisation est une zone de dévolatilisation en lit fluidisé.